# EUROPÄISCHE PATENTSCHRIFT

(11) **EP 4 074 721 B1**
(45) Veröffentlichungstag und Bekanntmachung des Hinweises auf die Patenterteilung: **04.10.2023**
(21) Anmeldenummer: 21168815.5
(22) Anmeldetag: 16.04.2021
(51) Int. Cl.: C07F 9/6574, C07C 45/50

(54) **DIPHOSPHITE MIT EINEM OFFENEN UND EINEM GESCHLOSSENEN 2,4-METHYLIERTEN FLÜGELBAUSTEIN**
DIPHOSPHITES HAVING ONE OPEN AND ONE CLOSED 2,4-METHYLATED BUILDING BLOCK
DIPHOSPHITE DOTÉ DES ÉLÉMENTS STRUCTURAUX 2,4-MÉTHYLÉS OUVERT ET FERMÉ

(43) Veröffentlichungstag der Anmeldung: 19.10.2022
(73) Patentinhaber: Evonik Operations GmbH, 45128 Essen (DE)
(72) Erfinder: FRANKE, Robert, 45772 Marl (DE); SALE, Anna Chiara, 45657 Recklinghausen (DE); BRÄCHER, Alexander, 45721 Haltern am See (DE); FRIDAG, Dirk, 45721 Haltern am See (DE); MARKOVIC, Ana, 45721 Haltern am See (DE); KUCMIERCZYK, Peter, 44628 Herne (DE); KNOSSALLA, Johannes, 46514 Gahlen (DE)
(74) Vertreter: Evonik Patent Association

(56) Entgegenhaltungen:
- EP-A2- 0 213 639

## Beschreibung

Die Erfindung betrifft Diphosphite mit einem offenen und einem geschlossenen 2,4-methylierten Flügelbaustein und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In EP 0213639 A2 ist auf Seite 98 in Beispiel 10 die folgende Verbindung dargestellt:

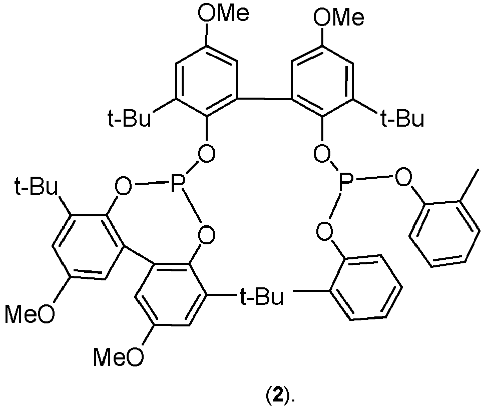

Die Verbindung (2) wird hier als Ligand in der Hydroformylierung von 1-Buten eingesetzt.

Die technische Aufgabe der Erfindung ist die Bereitstellung neuer Liganden, welche in der Hydroformylierung von Olefinen eine gesteigerte n/iso-Selektivität verglichen zu dem aus dem Stand der Technik bekennten Liganden aufweisen.

Die Aufgabe wird gelöst durch eine Verbindung gemäß Anspruch 1.

Verbindung gemäß der Struktur (I): 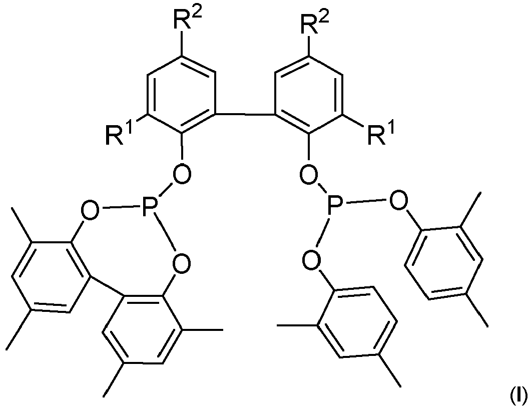 wobei R¹, R² ausgewählt sind aus: -H, -(C₁-C₁₂)-Alkyl, -O-(C₁-C₁₂)-Alkyl.

In einer Ausführungsform sind R¹, R² ausgewählt aus: -H, -(C₁-C₁₂)-Alkyl.

In einer Ausführungsform stehen R¹, R² für-(C₁-C₁₂)-Alkyl.

In einer Ausführungsform stehen R¹, R² für den gleichen Rest.

In einer Ausführungsform weist die Verbindung die Struktur (1) auf:

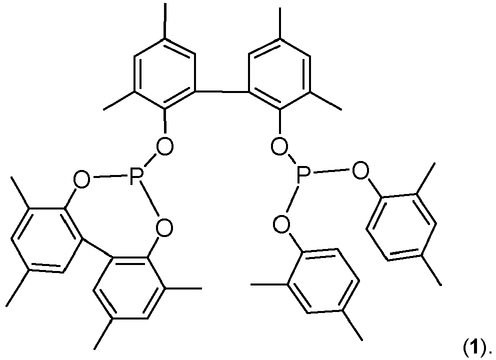

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 10 bis 60 bar. Besonders bevorzugt sind eine Temperatur von 100 °C bis 140 °C und ein Druck von 20 bis 50 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende Cs-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende Cs-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei derTetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Synthese

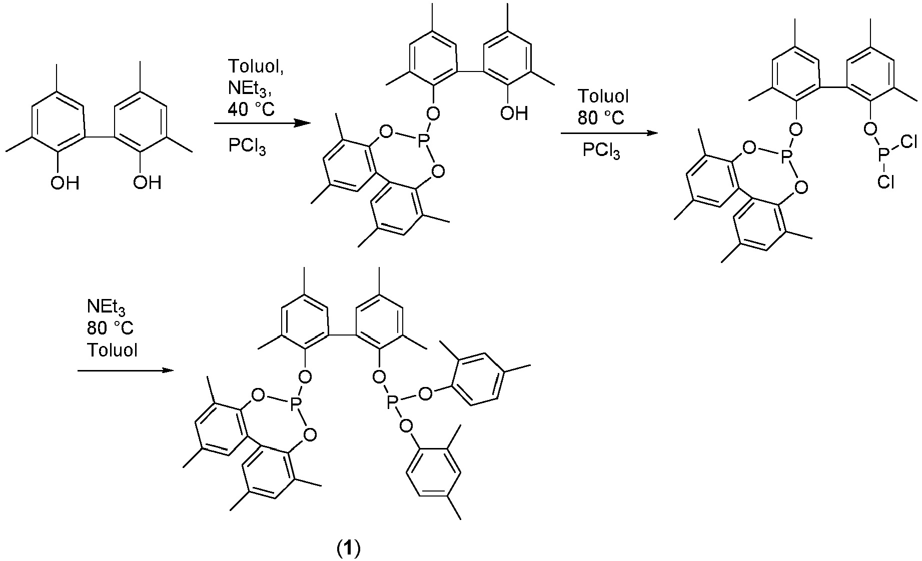

### Synthese (1a)

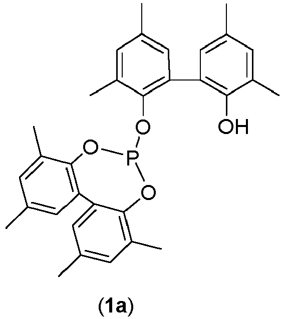

In einem 500 ml Schlenk mit Wellenbrecher wurden 10,7 g 2,2'-Bis(3,5-di-methyl)-phenol (Ölpumpenvakuum nachgetrocknet) vorgelegt in 100 mL Toluol gelöst und auf 40 °C erwärmt. In der Glovebox wurden 15,5 g Chlorophosphit in einem 250 ml Schlenk eingewogen und ausgeschleust. Das Chlorophosphit wurde unter Rühren in 150 ml Toluol gelöst und mit 5,6 mL EtsN versetzt. Die Chlorophosphit-EtsN-Toluol Lösung wurde innerhalb von 4 h bei 40 °C langsam zur vorgelegten Phenol-Lösung gegeben. Nach Rühren über Nacht wurde erneut 2,8 mL EtsN hinzugegeben. Nach 18 Stunden wurden 100 mL entgastes Acetonitril unter Rühren zum Feststoff gegeben. Die Mixtur wurde über Nacht bei Raumtemperatur gerührt. Am Morgen wurde die Mixtur auf 0 °C abgekühlt und 2 h bei dieser Temperatur gerührt. Dann wurde abgefrittet und mit wenig entgastem, kaltem Acetonitril nachgewaschen. Der Feststoff auf der Fritte wurde getrocknet und in die Glove-Bos eingeschleust. Reinheit: 95%, Ausbeute 74%.

### Synthese (1b)

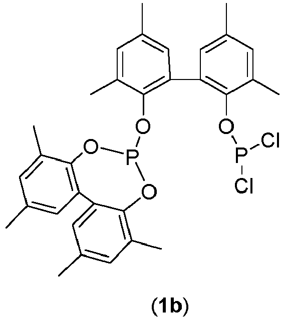

In einen sekurierten 250ml Schlenk werden nach dem Belüften mit Argon 50mL Toluol und 2,75 mL (0.062 mol) Phosphortrichlorid gegeben. In der Glovebox werden 6 g (0.02mol) (**1a**) in einen weiteren 250 ml Schlenk eingewogen. Nach dem Ausschleusen werden unter Argon 50 mL Toluol und 4,25 mL (0.061 mol) Triethyamin hinzugegeben. Nach dem vollständigen Lösen wird die (**1a**)-Lösung langsam zur Phosphortrichlorid/Toluol-Lösung getropft. Anschließend wurde die Lösung 2 Stunden bei 80°C gerührt, danach auf Raumtemperatur abgekühlt und über Nacht bei Raumtemperatur gerührt. Zur Aufarbeitung wird die Suspension am nächsten Morgen über eine G3-Fritte abfiltriert. Das hierbei abfiltrierte Hydrochlorid wird verworfen. Die erhaltene Mutterlauge wird mittels Ölpumpenvakuum bei 45°C bis zur Trockne eingeengt.

Ausbeute: 90%, Reinheit: 94,5 %.

### Synthese (1)

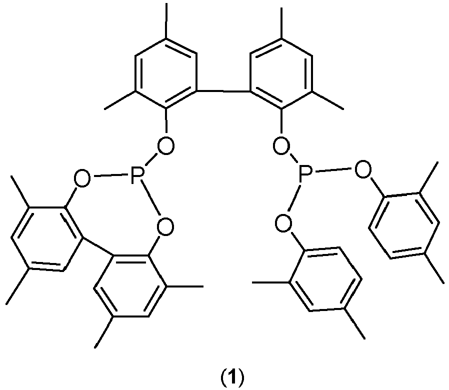

In der Glove-Box wurde in einem sekurierten 250 ml Schlenk 7.89 g (0,011 mol) Diorganophosphitdichlorphosphit (**1b**) abgewogen, anschließend ausgeschleusst und in 80 mL getrocknetem Toluol gelöst. In einem zweiten sekurierten 250 ml Schlenk wurden mittels Argon gespülter Spritze 2.64 ml (2.69 g = 0,022 mol) 2.4-Dimethylphenol befüllt. Anschließend wurde unter rühren 60 mL getrocknetes Toluol und 6.6 mL = 4,8 g (0,047 mol) entgastes Triethylamin zugegeben und unter Rühren aufgelöst. Zur Phenol-Triethylamin Lösung wurde dann die zuvor hergestellte Chlorophosphit-Lösung in einem Zug hinzugegeben. Im Anschluß wurde die Reaktionsmixtur sofort auf 80 °C erwärmt und kräftig über Nacht bei dieser Reaktionstemperatur gerührt. Zur Aufarbeitung wurde das entstandene Aminhydrochlorid bei Raumtemperatur abgefrittet. Zur besseren Filtrierbarkeit des Aminhydrochlorid wurde zuvor der Rührer abgestellt und die Reaktionsmischung 1,5 h stehen gelassen: Das erhaltene Filtrat wurde bis zur Trockne eingeengt und über 18 Stunden mittel Ölpumpenvakuum bei Raumtemperatur nachgetrocknet. Anschließend wurde der Rückstand mit 100 mL entgastem Acetonitril gerührt, er ging nicht in Lösung, daraufhin wurde das Lösemittel wieder abgezogen. Den dabei erhaltenen Feststoff wurde mit 100 mL entgastes N-Heptan versetzt. Dabei löste sich der überwiegende Teil in Heptan auf. Die getrübte Lösung wurde gefrittet. Das klare Filtrat bis zur Trockne eingeengt. Reinhet: 76,6%.

Um eine saubere Aufreinigung zu erzielen, wurde ein DC mit in verschiedenen Laufmittel durchgeführt. Als bestes Laufmittel erwies sich das Gemisch n-Hptan / Ethyacetat 96:4. Das Produkt wurde in 15 ml n-Heptan gelöst und flüssig in den Säulenautomaten beladen. Dazu wurde eine 120 g Kieselgelsäule genutzt. Mit Hilfe vom ermittelten Rf-Werten wurde ein Gradient vom Automaten ermittelt. Reinheit 95%, Ausbeute: 30%.

### Synthese (2) (Vergleichsligand)

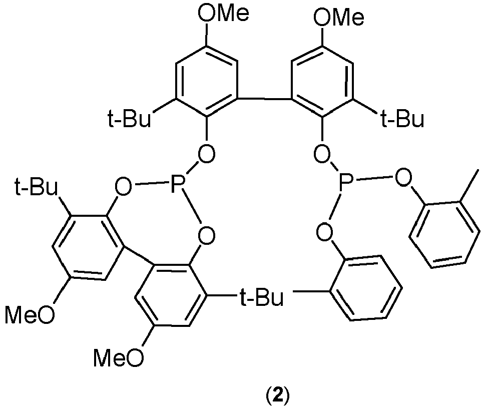

In der Glove-Box wurde in einem sekurierten 250 mL Schlenk 9 g (0,01 mol) Diorganophosphitdichlorphosphit eingewogen, anschließend ausgeschleusst und in 75 mL getrocknetem Toluol gelöst. In einem zweiten sekurierten 250 mL Schlenk wurden 2.2 g (2,1 mL 0,02 mol) 2-Methylphenol abgewogen und 12 h mittels Ölpumpenvakuum bei Raumtemperatur nachgetrocknet. Es wurde unter Rühren 50 mL getrocknetes Toluol und 3 mL = 2,2 g (0,022 mol) entgastes Triethylamin zugegeben und aufgelöst. Zur Phenol-Triethylamin Lösung wurde bei Raumtemperatur innerhalb 1,5 h das Dichlorophosphit hinzugegeben. Die Reaktionsmischung wurde 2 h bei Raumtemperatur gerührt und danach auf 80 °C erhitzt. Die Reaktionsmischung wurde 15 h bei dieser Temperatur gerührt und es wurden 3 mal 1,5 mL (0,011 mol) Triethylamin nachdosiert und 15 h weiter rühren gelassen. Das Ammoniumhydrochlorid wurde abgefrittet, 1 x mit 10 mL getrocknetem Toluol nachgewaschen bis zur Trockne eingeengt. Der Feststoff wurde 15 h bei Raumtemperatur getrocknet und mit 40 mL entgastem Acetonitril gerührt. Der ausgefallene weiße Feststoff wurde abgefrittet, der Schlenk wurde mit 2 mal 10 mL ACN nachgespült und nach dem Trocknen in die Glove-Box eingeschleust. Ausbeute 90%, Reinheit: 95%.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das als Substrat eingesetzte n-Octen (Oxeno GmbH, Octenisomerengemisch aus 1-Octen: 3 %; cis+trans-2-Octen: 49 %; cis+trans-3-Octen: 29 %; cis+trans-4-Octen: 16 %; gerüstisomere Octene: 3 %) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 0,0021 g Rh(acac)(CO)₂ und die entsprechende Menge an Phosphitverbindung eingewogen und mit 8,0 ml Toluol aufgefüllt. Die jeweils eingebrachte Masse an Toluol wurde für die GC-Analyse bestimmt. Anschließend wurden 1,80 g n-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %) : CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 20 bar erhöht und die Reaktion bei konstantem Druck für 4 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m x 0,2 mm x 0,5 µm. Die quantitative Bestimmung von Restolefin und Aldehyd erfolgte gegen das Lösungsmittel Toluol als interner Standard.

### Ergebnisse der Katalyseversuche

### Reaktionsbedingungen:

[Rh]: 120 ppm, L:Rh = 1:2, p: 20 bar, T: 120 °C; t: 4 h

**Tabelle 1: Hydroformylierung der n-Octene**

| Ligand | n/iso-Selektivität in % |
|---|---|
| **1*** | 77 |
| **2** | 56 |

| | |
|---|---|
| * erfindungsgemäße Verbindung | |

### Definition der Selektivität:

Bei der Hydroformylierung gibt es die n/iso-Selektivität: das Verhältnis von linearem Aldehyd (= n) zu verzweigtem Aldehyd (= iso). Hierbei bedeutet die Selektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Mit der erfindungsgemäßen Verbindung (**1**) konnte eine gegenüber dem Vergleichsliganden (**2**) gesteigerte n/iso-Selektivität erzielt werden.

Die durchgeführten Versuche belegen, dass die gestellte Aufgabe durch die erfindungsgemäße Verbindung gelöst wird.

## Patentansprüche

1. Verbindung gemäß der Struktur (I): 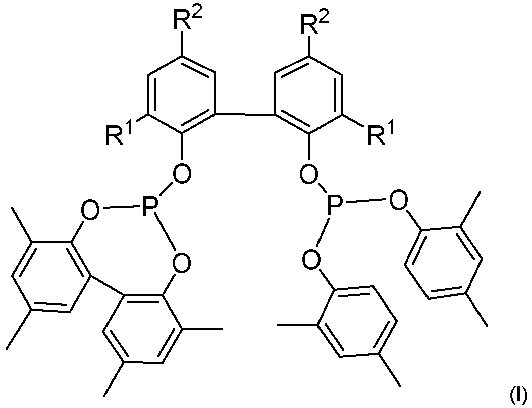 wobei R¹, R² ausgewählt sind aus: -H, -(C₁-C₁₂)-Alkyl, -O-(C₁-C₁₂)-Alkyl.

2. Verbindung nach Anspruch 1,
wobei R¹, R² ausgewählt sind aus: -H, -(C₁-C₁₂)-Alkyl.

3. Verbindung nach einem der Ansprüche 1 oder 2, wobei R¹, R² für-(C₁-C₁₂)-Alkyl stehen.

4. Verbindung nach einem der Ansprüche 1 bis 3, wobei R¹, R² für den gleichen Rest stehen.

5. Verbindung nach einem der Ansprüche 1 bis 4, wobei die Verbindung die Struktur (**1**) aufweist:

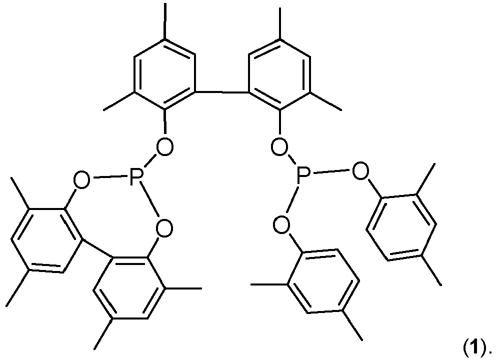

6. Verwendung einer Verbindung nach einem der Ansprüche 1 bis 5,
in einem Ligand-Metall-Komplex zur Katalyse einer Hydroformylierungsreaktion.

7. Verfahren umfassend die Verfahrensschritte:
a) Vorlegen eines Olefins,
b) Zugabe einer Verbindung nach einem der Ansprüche 1 bis 5 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

## Claims

1. Compound of the structure (**I**): 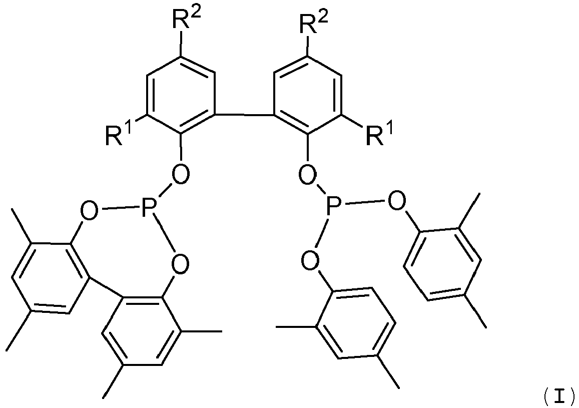 where R¹, R² are selected from: -H, - (C₁-C₁₂) -alkyl, -O-(C₁-C₁₂)-alkyl .

2. Compound according to Claim 1,
where R¹, R² are selected from: -H, -(C₁-C₁₂)-alkyl.

3. Compound according to either of Claims 1 and 2, where R¹, R² are -(C₁-C₁₂)-alkyl.

4. Compound according to any of Claims 1 to 3, where R¹, R² are the same radical.

5. Compound according to any of Claims 1 to 4, where the compound has the structure (**1**):

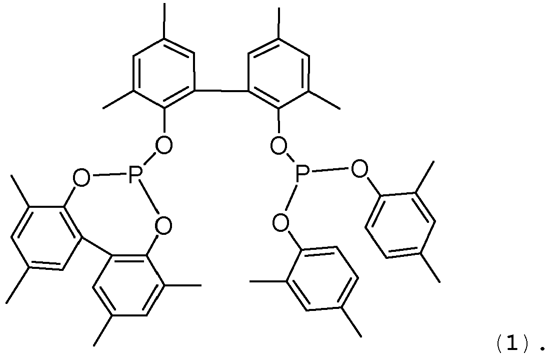

6. Use of a compound according to any of Claims 1 to 5 in a ligand-metal complex for catalysis of a hydroformylation reaction.

7. Process comprising the process steps of:
a) initially charging an olefin,
b) adding a compound according to any of Claims 1 to 5 and a substance comprising a metal selected from: Rh, Ru, Co, Ir,
c) supplying H₂ and CO,
d) heating the reaction mixture from a) to c), with conversion of the olefin to an aldehyde.

## Revendications

1. Composé selon la structure (**I**) : 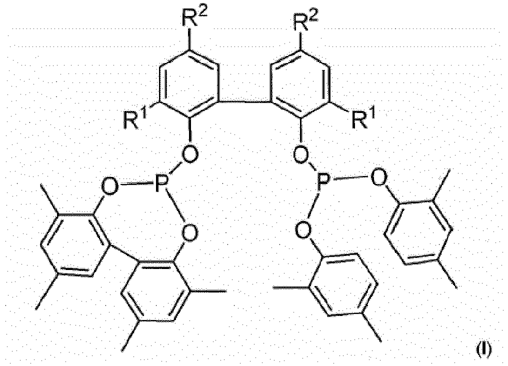 dans laquelle R¹, R² sont choisis parmi : -H, -alkyle (C₁-C₁₂), -O-alkyle (C₁-C₁₂).

2. Composé selon la revendication 1,
dans lequel R¹, R² sont choisis parmi : -H, -alkyle (C₁-C₁₂).

3. Composé selon l'une des revendications 1 et 2, dans lequel R¹, R² représentent -alkyle (C₁-C₁₂).

4. Composé selon l'une des revendications 1 à 3, dans lequel R¹, R² représentent le même radical.

5. Composé selon l'une des revendications 1 à 4, dans lequel le composé présente la structure (**1**) :

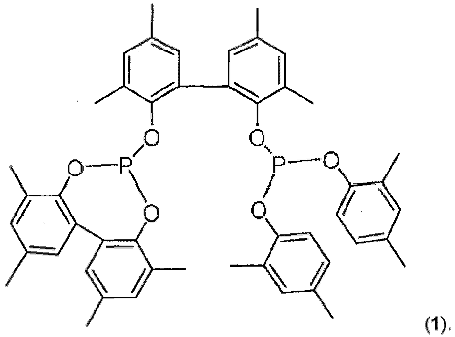

6. Utilisation d'un composé selon l'une des revendications 1 à 5,
dans un complexe ligand-métal pour la catalyse d'une réaction d'hydroformylation.

7. Procédé comprenant les étapes de processus :
a) disposition au préalable d'une oléfine,
b) addition d'un composé selon l'une des revendications 1 à 5 et d'une substance qui comporte un métal choisi parmi : Rh, Ru, Co, Ir,
c) introduction de H₂ et CO,
d) chauffage du mélange réactionnel provenant de a) à c), lors duquel l'oléfine est convertie en un aldéhyde.
